# EUROPEAN PATENT SPECIFICATION

(11) **EP 4 329 867 B1**
(45) Date of publication and mention of the grant of the patent: **17.09.2025**
(21) Application number: 21733594.2
(22) Date of filing: 07.05.2021
(51) Int. Cl.: A61M 39/02, A61B 17/3209, A61M 25/01, A61B 17/32

(54) **LOW PROFILE, INJECTABLE SUBCUTANEOUS VASCULAR ACCESS SYSTEM**
SYSTEM FÜR SUBKUTANEN GEFÄSSZUGANG MIT NIEDRIGEM PROFIL
SYSTÈME D'ACCÈS VASCULAIRE SOUS-CUTANÉ INJECTABLE À PROFIL RÉDUIT

(43) Date of publication of application: 06.03.2024
(73) Proprietor: Bard Peripheral Vascular, Inc., Franklin Lakes, NJ 07417 (US)
(72) Inventor: DENSLEY, Bryon Ray, Fremont, CA 94538 (US); ANDERSEN, Christian, Kaysville, UT 84037 (US)
(74) Representative: Hoffmann Eitle
(86) International application number: PCT/US2021/031407
(87) International publication number: WO 2022/235276

(56) References cited:
- WO-A1-2020/028847
- WO-A1-2021/078488
- US-A- 5 281 199
- US-A1- 2005 203 484
- US-A1- 2008 249 509
- US-A1- 2009 076 466
- US-A1- 2011 034 886

## Description

### BACKGROUND

WO 2021/078488 A1 discloses an implantable access device for accessing the vascular system of a human or animal body, particularly subcutaneously implantable access port, comprising a port body, a needle entrance with at least one inlet opening for receiving a needle and an outlet opening, an at least partially flexible catheter for accessing the vascular system of the human or animal body connected to the outlet opening of the needle entrance wherein the needle entrance is connected to the port body and movable relative to the port body between a first, unactuated operating condition and a second, actuated operating condition, so that in the first, unactuated operating condition the clamping means prevent a fluid flow through the at least partially flexible catheter and in the second, actuated operating condition allow a fluid flow through the at least partially flexible catheter.

US 5281199 A discloses an implantable access device.

US 2005/203484 A1 discloses a multi-functional port.

US 2008/249509 A1 discloses a stabilized elongate implantable vascular access device.

US 2011/034886 A1 discloses an implantable medical device too and method of use.

US 2009/076466 A1 discloses a sutureless venous access port.

WO 2020/028847 A1 discloses an implantable port placement system including low-scarring exterior.

### SUMMARY

Briefly summarized, embodiments disclosed herein are directed to a low profile, injectable subcutaneous vascular access system and associated methods thereof. The system includes a low profile port, defining an outer profile that is substantially the same as an outer profile of a catheter coupled thereto. The low-profile port can be placed subcutaneously and requires a much smaller insertion site that conventional ports. As such fewer stiches, or no stitches, are required to close the insertion site similar to that of an injection, making the port "injectable" and improving patient recovery times, reducing scaring, and improving aesthetics. Further, a lumen of the port can define the same diameter or cross-sectional shape as that of the catheter lumen, and can align with the catheter lumen, providing little to no resistance in fluid flow therethrough. Also disclosed are placement tools configured to form an insertion site and place the port subcutaneously.

Disclosed herein is a vascular access device configured to be disposed subcutaneously including, a catheter defining a catheter lumen and providing fluid communication with a vasculature of a patient and a port including a body defining a port lumen extending along a longitudinal axis from an inlet to an outlet, the port lumen defining a uniform cross-sectional diameter between the inlet and the outlet.

In some embodiments, the port lumen defines a uniform cross-sectional area. In some embodiments, the port lumen defines a uniform cross-sectional shape. In some embodiments, the port lumen includes a valve or a needle penetrable septum configured to control a fluid flow therethrough. In some embodiments, the outlet of the port is defined by a stem configured to be inserted into the lumen of the catheter. In some embodiments, the diameter of the port lumen is equal to a diameter of the lumen of the catheter in the relaxed state. In some embodiments, the cross-sectional area of the port lumen is equal to a cross-sectional area of the lumen of the catheter in the relaxed state.

In some embodiments, the cross-sectional shape of the port lumen is equal to a cross-sectional shape of the lumen of the catheter in the relaxed state. In some embodiments, the vascular access device further includes a nub extending proximally from a lower edge of the inlet and configured to receive an access needle, impinging thereon, and direct the needle towards the inlet. In some embodiments, the nub includes a side wall extending perpendicular from an edge of the nub and extending longitudinally, the side wall configured to direct a needle impinging thereon towards the inlet. In some embodiments, an axis of a portion of the port lumen aligns with an axis of the catheter lumen. In some embodiments, the portion of the port lumen extends between a septum and the outlet.

In some embodiments, an axis of a second portion of the port lumen extends at an angle relative to the first portion, the second portion disposed proximal of the septum. In some embodiments, an outer diameter of the port body is less than double an outer diameter of the catheter. In some embodiments, an outer diameter of the port body is between than 170% and 130% an outer diameter of the catheter. In some embodiments, the port body includes an echogenic or radiopaque material. In some embodiments, the vascular access device further includes an insertion tool including a tool head having a sharpened front edge and configured to form a tissue pocket to receive the vascular access device. In some embodiments, the vascular access device further includes a cathlock configured to secure the catheter to the outlet.

Also disclosed is a port placement system according to the invention, configured for placing a port subcutaneously including, a port defining a lumen extending along a longitudinal axis from an inlet to an outlet, the lumen defining a uniform cross-sectional diameter between the inlet and the outlet, a catheter coupled to and in fluid communication with the distal outlet, and a placement tool including, a housing defining a cavity configured to receive the port therein, and including an elongate opening extending longitudinally along a bottom surface thereof and communicating with the cavity, a handle extending from the housing, and a nose portion hingedly coupled to the housing at an opposite end from the handle, and configured to transition between a closed position and an open position to release the port from the cavity.

In some embodiments, the nose portion defines a tapered outer profile. In some embodiments, the placement tool includes a sharpened front edge configured to form one of an incision site or a tissue pocket. In some embodiments, the port placement system further includes a blade disposed in the front edge. In some embodiments, the port placement system further includes an actuator button disposed on the handle and configured to transition the nose portion between the open position and the closed position. In some embodiments, a lateral width of the elongate opening is less than a diameter of the port and greater than a diameter of the catheter.

In some embodiments, the handle defines a cavity and an elongate opening, the handle cavity communicating with the cavity of the housing and the handle elongate opening communicating with the elongate opening of the housing. In some embodiments, a lateral width of the handle elongate opening is larger than a diameter of the port. In some embodiments, the lumen of the port defines a uniform cross-sectional area. In some embodiments, the lumen of the port defines a uniform cross-sectional shape. In some embodiments, the lumen of the port includes a valve or a needle penetrable septum.

In some embodiments, the outlet of the port is defined by a stem configured to be inserted into a lumen of the catheter. In some embodiments, the port placement system further includes a cathlock configured to secure the catheter to the port. In some embodiments, the port further includes a nub extending from a bottom edge of the inlet and configured to receive an access needle, impinging thereon, and direct the needle towards the inlet. In some embodiments, the port includes an echogenic or radiopaque material. In some embodiments, an axis of the lumen aligns with an axis of the catheter.

Also disclosed is a method of placing a port having a catheter coupled thereto including, disposing the port within a housing of an insertion tool, the tool including an elongate opening extending along a bottom surface of the housing and communicating with a cavity of the housing, urging a front edge the tool subcutaneously, forming a tissue pocket to receive the port, transitioning a nose portion of the housing to an open position, withdrawing the insertion tool proximally, and disengaging the tool from the port and the catheter.

In some embodiments, the elongate opening of the housing defines a lateral width that is less than a diameter of the port and greater than a diameter of the catheter. In some embodiments, the method further includes a handle extending from the housing opposite from the front edge. In some embodiments, the handle defines a handle cavity communicating with the cavity of the housing. In some embodiments, the handle includes an elongate opening extending along a lower surface thereof and communicating with the elongate opening of the housing. In some embodiments, a lateral width of the elongate opening of the handle is greater than a diameter of the port.

In some embodiments, the method further includes forming an incision site with the front edge of the insertion tool. In some embodiments, the method further includes actuating an actuator disposed on the tool to transition the nose portion between the closed position and the open position. In some embodiments, disengaging the tool from the port and the catheter further includes sliding the catheter through the elongate opening of the housing.

### DRAWINGS

A more particular description of the present disclosure will be rendered by reference to specific embodiments thereof that are illustrated in the appended drawings. It is appreciated that these drawings depict only typical embodiments of the invention and are therefore not to be considered limiting of its scope. Example embodiments of the invention will be described and explained with additional specificity and detail through the use of the accompanying drawings in which:
FIG. 1A shows a perspective view of a low profile, injectable port, in accordance with embodiments disclosed herein.
FIG. 1B shows a side view of a low profile, injectable port, in accordance with embodiments disclosed herein.
FIG. 1C shows a side view of a low profile, injectable port including a nub, in accordance with embodiments disclosed herein.
FIG. 2A shows a perspective view of a low profile, injectable port, in accordance with embodiments disclosed herein.
FIG. 2B shows a side view of a low profile, injectable port, in accordance with embodiments disclosed herein.
FIG. 3A shows a perspective view of an insertion tool, in accordance with embodiments disclosed herein.
FIG. 3B shows close up detail of the insertion tool of FIG. 3A, in accordance with embodiments disclosed herein.
FIG. 3C shows a top view of an insertion tool, in accordance with embodiments disclosed herein.
FIG. 3D shows a side view of an insertion tool, in accordance with embodiments disclosed herein.
FIG. 4A shows a perspective view of a port placement system, in accordance with the invention.
FIG. 4B shows close up detail of the port placement system of FIG. 4A, in accordance with the invention.
FIG. 4C shows a top view of a port placement system, in accordance with the invention.
FIG. 4D shows a side view of a port placement system, in accordance with the invention.
FIG. 4E shows a bottom view of a port placement system, in accordance with the invention.

### DESCRIPTION

Before some particular embodiments are disclosed in greater detail, it should be understood that the particular embodiments disclosed herein do not limit the scope of the concepts provided herein. It should also be understood that a particular embodiment disclosed herein can have features that can be readily separated from the particular embodiment and optionally combined with or substituted for features of any of a number of other embodiments disclosed herein.

Regarding terms used herein, it should also be understood the terms are for the purpose of describing some particular embodiments, and the terms do not limit the scope of the concepts provided herein. Ordinal numbers (e.g., first, second, third, etc.) are generally used to distinguish or identify different features or steps in a group of features or steps, and do not supply a serial or numerical limitation. For example, "first," "second," and "third" features or steps need not necessarily appear in that order, and the particular embodiments including such features or steps need not necessarily be limited to the three features or steps. Labels such as "left," "right," "top," "bottom," "front," "back," and the like are used for convenience and are not intended to imply, for example, any particular fixed location, orientation, or direction. Instead, such labels are used to reflect, for example, relative location, orientation, or directions. Singular forms of "a," "an," and "the" include plural references unless the context clearly dictates otherwise.

With respect to "proximal," a "proximal portion" or a "proximal end portion" of, for example, a catheter disclosed herein includes a portion of the catheter intended to be near a clinician when the catheter is used on a patient. Likewise, a "proximal length" of, for example, the catheter includes a length of the catheter intended to be near the clinician when the catheter is used on the patient. A "proximal end" of, for example, the catheter includes an end of the catheter intended to be near the clinician when the catheter is used on the patient. The proximal portion, the proximal end portion, or the proximal length of the catheter can include the proximal end of the catheter; however, the proximal portion, the proximal end portion, or the proximal length of the catheter need not include the proximal end of the catheter. That is, unless context suggests otherwise, the proximal portion, the proximal end portion, or the proximal length of the catheter is not a terminal portion or terminal length of the catheter.

With respect to "distal," a "distal portion" or a "distal end portion" of, for example, a catheter disclosed herein includes a portion of the catheter intended to be near or in a patient when the catheter is used on the patient. Likewise, a "distal length" of, for example, the catheter includes a length of the catheter intended to be near or in the patient when the catheter is used on the patient. A "distal end" of, for example, the catheter includes an end of the catheter intended to be near or in the patient when the catheter is used on the patient. The distal portion, the distal end portion, or the distal length of the catheter can include the distal end of the catheter; however, the distal portion, the distal end portion, or the distal length of the catheter need not include the distal end of the catheter. That is, unless context suggests otherwise, the distal portion, the distal end portion, or the distal length of the catheter is not a terminal portion or terminal length of the catheter.

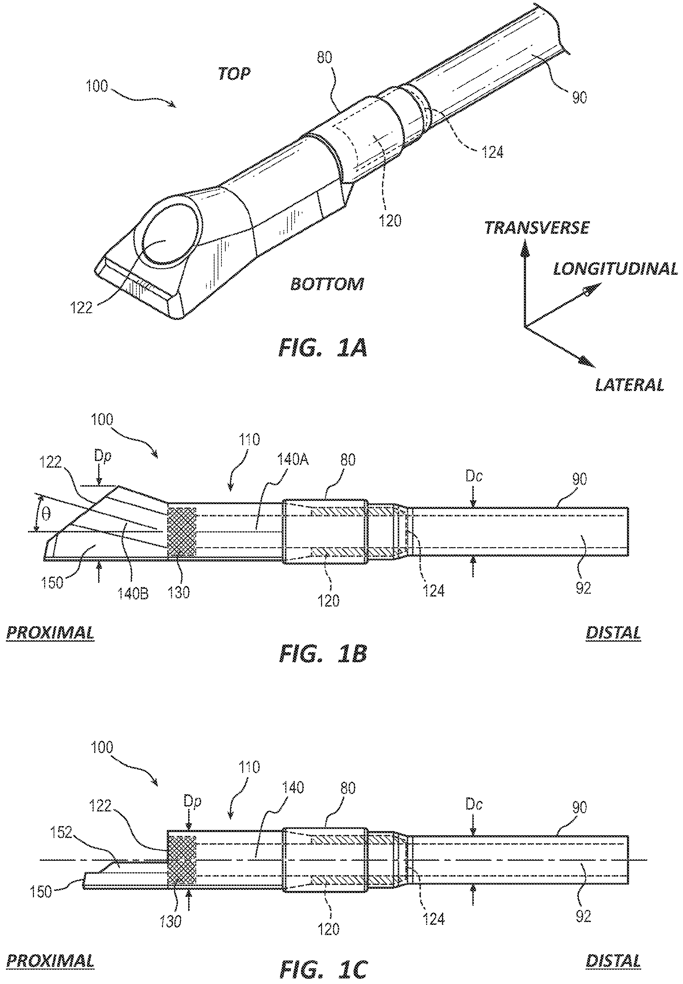

To assist in the description of embodiments described herein, as shown in FIG. 1A, a longitudinal axis extends substantially parallel to an axial length of the catheter. A lateral axis extends normal to the longitudinal axis, and a transverse axis extends normal to both the longitudinal and lateral axes.

Unless defined otherwise, all technical and scientific terms used herein have the same meaning as commonly understood by those of ordinary skill in the art.

FIGS. 1A-1C show a low profile, injectable vascular access device, or "port" 100, configured to be disposed subcutaneously and accessed by a needle, cannula or similar device, to provide fluid communication with a vasculature of a patient. FIG. 1A shows a perspective view of the port 100. FIGS. 1B-1C show side views of embodiments of a port 100. The port 100 generally includes a port body 110 extending axially, and a stem 120 extending therefrom and configured to engage a catheter 90. As used herein a port 100 can be disposed towards the proximal end of the port100 / catheter 90 assembly, and the catheter can be disposed towards the distal end of the port 100 / catheter 90 assembly.

The catheter 90 can include a compliant elongate tube, or body, configured to engage the port stem 120 in an interference fit. A distal end of the catheter 90 can be disposed within a vasculature of a patient to provide fluid communication therewith. A proximal end of the catheter 90 can be configured to be stretched over the port stem 120 to provide a fluid-tight seal there between. Worded differently, the stem 120 can be configured to engage an inner lumen of the catheter 90. The catheter 90 can be elastically deformed to receive the port stem 120 therein. In a relaxed state, the catheter lumen 92 can define a diameter, a cross-sectional area, and a cross-sectional shape (e.g. circular, semi-circular, elliptical, or the like). In an embodiment, a cathlock 80 can engage an outer surface of the catheter 90 and can further secure the catheter 90 to the port stem 120.

In an embodiment, the port 100 can define a lumen 140 extending from an inlet 122 disposed at a proximal end, to an outlet 124 disposed at a distal end. In an embodiment, the outlet 124 can be defined by the port stem 120. In an embodiment, an inlet 122 can be defined as a proximal-most opening to the lumen 140 and can be configured to receive a needle therein. In an embodiment, the outlet 124 can be defined as a distal-most opening of the port lumen 140 and can provide fluid communication with the catheter lumen 92. In an embodiment, the port lumen 140 can define a substantially uniform diameter along the entire axial length thereof, i.e. from the inlet 122 to the outlet 124. In an embodiment, the port lumen 140 can define a substantially uniform lateral cross-sectional area along the entire axial length thereof. In an embodiment, the port lumen 140 can define a substantially uniform lateral cross-sectional shape along the entire axial length thereof.

In an embodiment, a diameter of the port lumen 140 can be equal to a diameter of the catheter lumen 92. In an embodiment, a cross-sectional area of the port lumen 140 can be equal to a cross-sectional area of the catheter lumen 92. In an embodiment, a cross-sectional shape of the port lumen 140 can be equal to a cross-sectional shape of the catheter lumen 92. In an embodiment, an axis of the port lumen 140 can extend parallel to, or align with, an axis of the catheter lumen 92. In an embodiment, the port 100 can further include a needle-penetrable septum 130, valve, or similar structure configured to control a fluid flow through the lumen 140.

Advantageously, the port lumen 140 can provide a direct fluid path with the catheter lumen 92, with little to no variation is diameter, cross-sectional area, or cross-sectional shape, to minimize any fluid resistance therethrough. Further, an axis of the port lumen 140 can align with an axis of the catheter lumen 92 to minimize any fluid resistance therethrough. This allows for increased flow velocities through the port 100, through the catheter 90 and into the vasculature of the patient.

In an embodiment, an outer diameter or outer profile of the port body 110 can be substantially the same, or slightly larger than, an outer diameter or outer profile of the catheter 90. In an embodiment, the diameter of the port (*D_{P}*) at the widest or tallest point, can be less than double the diameter of the catheter (*D_{C}*)*.* Worded differently, a maximum diameter of the port (*D_{P}*) along an axis extending perpendicular to the longitudinal axis can be less than 200% of the diameter of the catheter (*D_{C}*) along the same axis. In an embodiment, the diameter of the port (*D_{P}*) can be between 130% and 170% of the diameter of the catheter (*D_{C}*)*.* Advantageously, the maximum dimensions of the port 110 can be substantially the same, or only slightly larger than the dimensions of the catheter 90. As such, placing the port 110 can require the same sized incision site as would be required to place the catheter 90 alone. Advantageously, the incision site required to place the port can require fewer stiches to close, or require no stitches to close the site, improving patient recovery times, reducing scaring and improving aesthetics.

In an embodiment, not forming part of the claimed invention, as shown in FIG. 1B, a first portion of the port lumen 140A, e.g. disposed between the septum 130 and the outlet 124, can extend along an axis that aligns with an axis of the catheter lumen 92. A second portion of the port lumen 140B, e.g. disposed proximal of the septum 130, can extend at an angle (*θ*) relative to the first portion of the port lumen 140A. The angle (*θ*) can be between 1° and 30°.

In an embodiment, as shown in FIG. 1C, the port body 110 can define a lumen 140 extending straight, along a longitudinal axis and aligned with an axis of the catheter lumen 92. In an embodiment, the port body 110 can further include a nub 150 extending from a bottom edge of the inlet 122 and can be configured to receive a needle tip, impinging thereon and guide the needle tip towards the inlet 122. The nub 150 can extend parallel with a bottom surface of the port body 110. In an embodiment, the nub 150 can define a substantially flat upper surface. In an embodiment, the nub 150 can include a concave upper surface. In an embodiment, the nub 150 can include a guide channel, or groove configured to receive the needle tip therein and guide the needle tip towards the inlet 122. In an embodiment, the nub 150 can include one or more side walls 152 extending longitudinally from a lateral edge of the nub 150 and configured to guide a needle towards the inlet 122. The side wall 152 can prevent a needle tip from sliding laterally, off of the upper surface of the nub 150.

In an embodiment, a lower surface of the port body 110 can define a substantially flat surface. In an embodiment, a portion of the port 110 can include an echogenic or radiopaque material configured to facilitate locating and accessing the port under medical imaging.

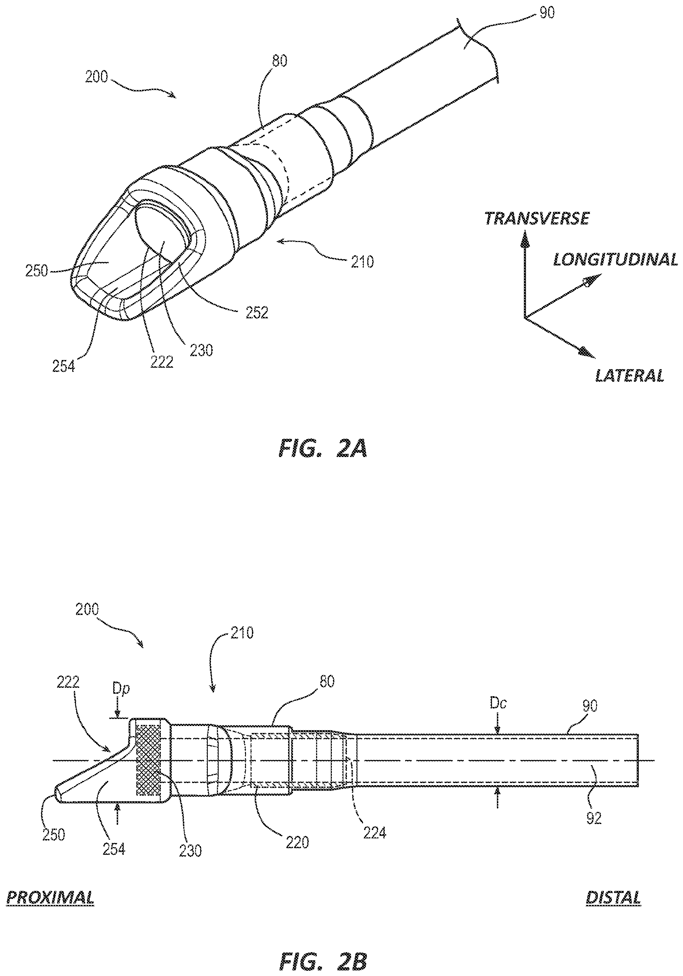

FIGS. 2A-2B show an embodiment of a low profile, injectable vascular access device, or "port" 200. FIG. 2A shows a perspective view of the port 200. FIG. 2B shows a side view of the port 200. The port 200 generally includes a port body 210 extending axially, and a stem 220 extending therefrom, defining a distal opening and configured to engage a catheter 90, as described herein. In an embodiment, a cathlock 80 can engage an outer surface of the catheter 90 and can further secure the catheter 90 to the port stem 220, as described herein.

In an embodiment, the port 200 can define a lumen 240 extending from an inlet 222 disposed at a proximal end, to an outlet 224 disposed at a distal end. In an embodiment, the outlet 224 can be defined by the port stem 220 of the port stem 220. In an embodiment, the port lumen 240 can define a substantially uniform diameter along the entire axial length thereof, i.e. from the inlet 222 to the outlet 224. In an embodiment, the port lumen 240 can define a substantially uniform cross-sectional area along the entire axial length thereof. In an embodiment, the port lumen 240 can define a substantially uniform lateral cross-sectional shape along the entire axial length thereof.

In an embodiment, a diameter of the port lumen 240 can be equal to a diameter of the catheter lumen 92. In an embodiment, a cross-sectional area of the port lumen 240 can be equal to a cross-sectional area of the catheter lumen 92. In an embodiment, a cross-sectional shape of the port lumen 240 can be equal to a cross-sectional shape of the catheter lumen 92. In an embodiment, an axis of the port lumen 240 can extend parallel to, or align with, an axis of the catheter lumen 92. In an embodiment, the port 200 can further include a needle-penetrable septum 230, valve, or similar structure configured to control a fluid flow therethrough.

Advantageously, the port lumen 240 can define a straight path with little to no variation is diameter, cross-sectional area, or cross-sectional shape, to minimize any fluid resistance therethrough. Further, the port lumen 240 can match the diameter, cross-sectional area, or cross-sectional shape of the catheter lumen 92 with little to no variation therebetween to minimize any fluid resistance between the port 200 and the catheter 90.

In an embodiment, an outer diameter or profile of the port body 210 can be the same or only slightly larger than an outer diameter or profile of the catheter 90. In an embodiment, the diameter of the port (*D_{P}*) at the widest or tallest point, can be less than double the diameter of the catheter (*D_{C}*)*.* Worded differently, a maximum diameter of the port (*D_{P}*) along an axis extending perpendicular to the longitudinal axis can be less than 200% of the diameter of the catheter (*D_{C}*) along the same axis. In an embodiment, the diameter of the port (*D_{P}*) can be between 130% and 170% of the diameter of the catheter (*D_{C}*)*.* Advantageously, the maximum dimensions of the port 210 can be substantially the same, or only slightly larger than the dimensions of the catheter 90. As such, placing the port 210 can require the same sized incision site as would be required to place the catheter 90 alone. Advantageously, the incision site required to place the port 200 can be substantially less than conventional ports and require fewer stiches to close, or require no stitches, to close the site. This can lead to improved recovery times, less scarring and improved aesthetics for the patient.

In an embodiment, the port 210 can include a nub 250 extending proximally from the port 200, i.e. away from the inlet 222 at an opposite end of the port 200 from the stem. The nub 250 can extend parallel with a bottom surface of the port body 210 and can be configured to guide a needle towards the inlet 222. Optionally, the nub 250 can include one or more side walls 252 angled and configured to guide a needle towards the septum 230. In an embodiment, the nub 250 can include a guide channel 254 extending longitudinally and configured to receive a tip of a needle therein and guide the needle tip towards the inlet 222. In an embodiment, a portion of the port 210 can include an echogenic or radiopaque material configured to facilitate locating and accessing the port under medical imaging.

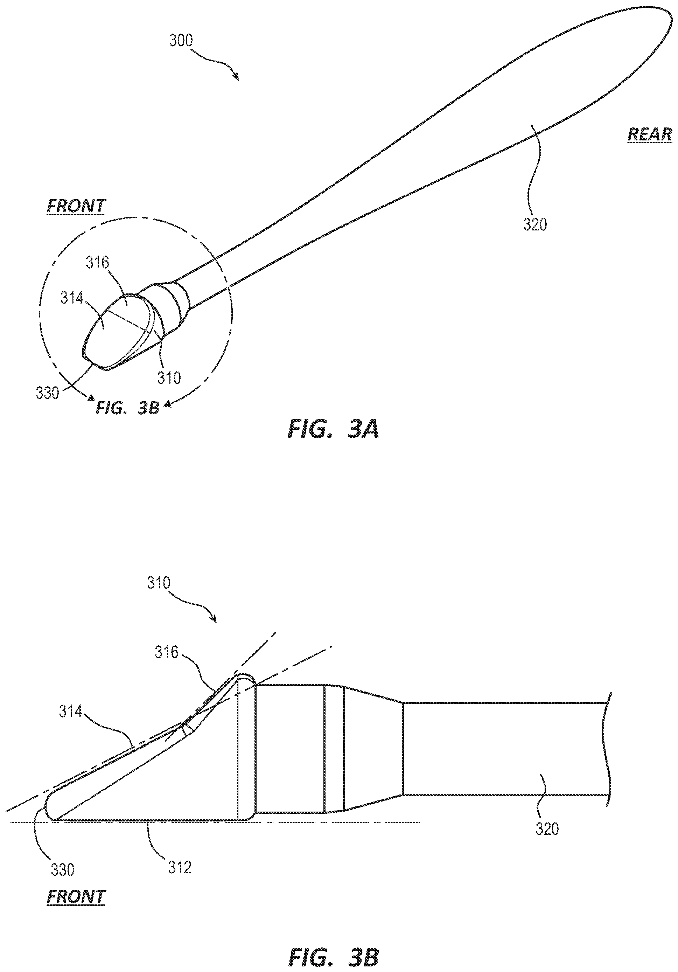

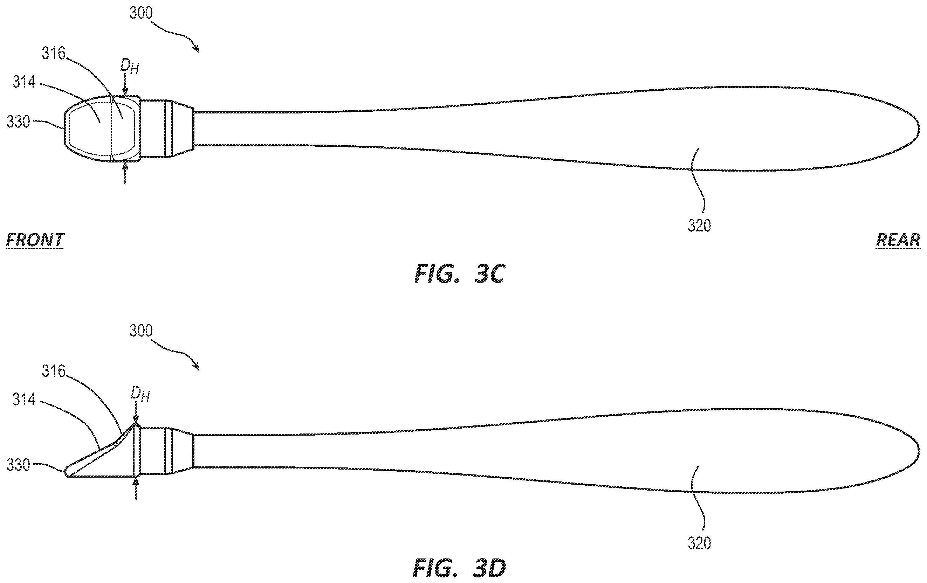

FIGS. 3A-3D show an embodiment of a placement tool 300 configured to form a tissue pocket for receiving a low-profile, injectable port 100, 200, as described herein. The tool 300 can generally include a tool head 310 disposed at a front end, and a handle 320 extending rearwards from the head 310. The handle 320 can be configured to be grasped by the clinician to manipulate the head 310 and form a tissue pocket.

In an embodiment, the head 310 can define a tapered shape extending longitudinally along one of the horizontal or vertical planes. In an embodiment the head 310 can define a spade-like shape. In an embodiment, the head 310 can define a profile that is similar in profile to the nub 150, 250 and or port body 110, 210 of the ports 100, 200. As such, as the head 310 is urged subcutaneously, the head 310 can define a tissue pocket that matches the shape, or outer profile, of the port 100, 200 minimizing subcutaneous travel.

In an embodiment, the head 310 can define a substantially flat lower surface 312 and a beveled front surface 314 each extending rearwards from a front edge 330. As shown in FIG. 3B, the beveled front surface 314 can extend at a first angle relative to the flat lower surface. In an embodiment, the head 310 can include a beveled upper surface 316 extending from the rear-most edge of the front surface 314 and extending at a second angle, different from the first angle, relative to the lower surface 312.

In an embodiment, the head 310 can include a sharpened front edge 330, configured to separate subcutaneous tissues to form a tissue pocket as the head 310 is urged forward, subcutaneously. In an embodiment, the front edge 330 can be configured to form an insertion site. In an embodiment, the front edge 330 can include a blade, or similar structure, formed of a different material from that of the head 310, and configured to form an insertion site and/or separate subcutaneous tissues to form a tissue pocket. In an embodiment, a largest transverse or lateral diameter of the head 310 (*D_{H}*) can be the same or slightly larger than the largest transverse or lateral diameter (*D_{P}*) of the port 100, 200.

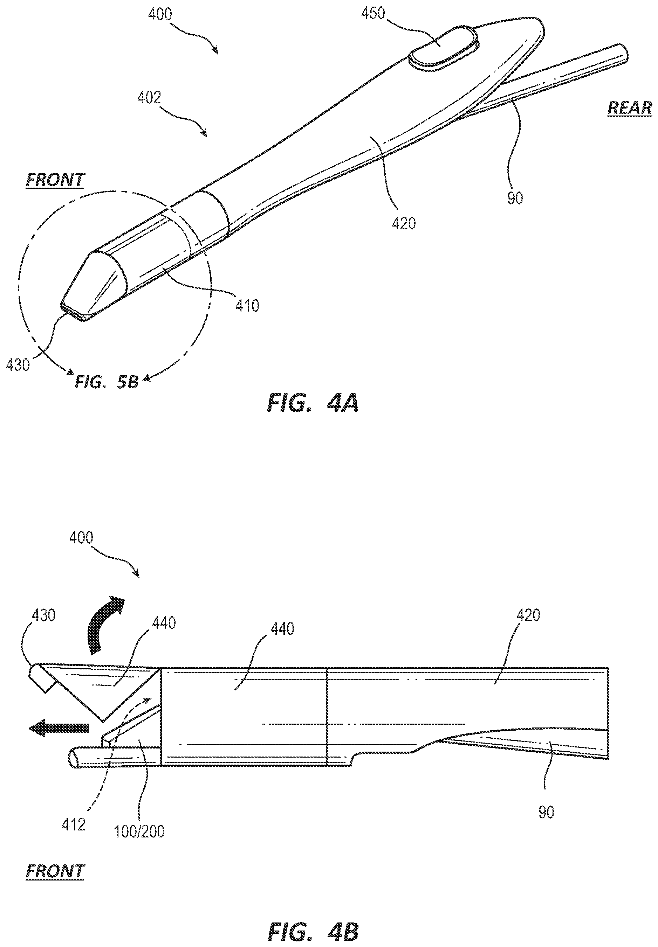

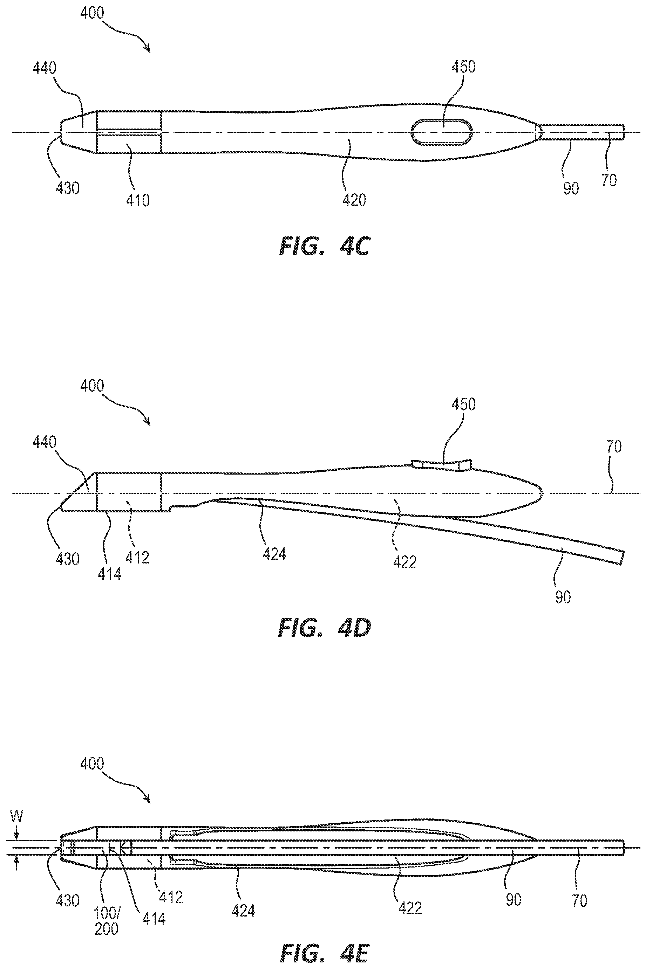

FIGS. 4A-4E show an embodiment of a port placement system 400. The system 400 can generally include a placement tool 402 configured to receive a low-profile, injectable port therein, e.g. port 100 or port 200, and place the port 100, 200 subcutaneously. The tool 402 can generally include a housing 410, and a handle 420 extending rearwards therefrom. The housing 410 can include an angled or sharpened front edge 430 configured to separate subcutaneous tissues to form a tissue pocket. The housing 410 can define a cavity 412 configured to receive a port 100, 200 therein. The housing 410 can further include an elongate opening 414 extending along a lower surface thereof and communicating with the cavity 412. In an embodiment, the elongate opening 414 can define a width (w) that is less than an outer-most diameter (*D_{P}*) of the port 100, 200 and can be greater or substantially the same as a diameter (*D_{C}*) of the catheter 90.

In an embodiment, the handle 420 can define a handle cavity 422 that communicates with the cavity 412 of the housing 410. The handle 420 can include an elongate opening 424 extending along a bottom surface and communicating with the handle cavity 422. The handle opening 424 can axially align with, and/or communicate with, the elongate opening 414 of the housing 410. The handle opening 424 can define a lateral width that is greater than an outer-most diameter (*D_{P}*) of the port 100, 200. As such, the handle opening 424 can allow a clinician to load a port 100, 200 and catheter 90 assembly into the housing cavity 412, to be retained therein, by passing the port 100, 200 through the handle opening 424 into the handle cavity 422 and advancing the port 100, 200 forwards into the cavity 412 of the housing 410.

In an embodiment, the housing 410 can include a nose portion 440 hingedly coupled to the housing 410 and transitionable between a closed position and an open position. In an embodiment, the nose portion 440 can define the front edge 430. In an embodiment, a portion of the housing 410 can define the front edge 430. In an embodiment, the nose portion 440 in the closed position can co-operate with a portion of the housing 410 to define the front edge 430. The nose portion 440 in the closed position (FIGS. 4A, 4C-4E) can define an angled, tapered, spade-like, or beveled profile extending rearwards from the front edge 430. The nose portion 440 can be configured to separate subcutaneous tissues to form a tissue pocket, as the tool 402 is urged subcutaneously, as described herein. In an embodiment, the front edge 430 can include a blade or similar structure configured to form an incision site and/or separate subcutaneous tissues to form a tissue pocket. In an embodiment, a profile of the nose portion 440 can substantially match the outer profile of the port 100, 200. This can provide a tissue pocket that matches the outer profile of the port 100, 200 and can minimize subcutaneous travel. In an embodiment, the nose portion 440 can pivot to an open position (FIG. 4B), to provide communication with the interior cavity 412 of the housing 410 and allow the port 100, 200 to slide distally and be placed within the tissue pocket.

In an embodiment, the handle 420 can include an actuator 450 functionally coupled with the nose portion 440 and configured to transition the nose portion between the open position and the closed position. In an embodiment, the actuator 450 can be a push button and include one or more gears, levers, biasing members, or similar mechanisms configured to transition the nose portion 440 between the open position and the closed position when the push button is actuated. It will be appreciated that the push button is exemplary and various sliders, dials, thumb wheels, levers, or similar actuators are contemplated.

In an exemplary method of use a port placement system 400 is provided generally including a tool 402 and a port, e.g. port 100, 200, as described herein. In an embodiment, the port 100, 200 can be loaded into the tool 402 by the clinician. In an embodiment, the port 100, 200 can be pre-loaded within the tool 402. The clinician can load the port 100, 200 into the tool 402 by passing the port 100, 200 through a handle opening 424 and into the handle cavity 422. A lateral width of the handle opening 424 can be wider than an outer diameter (*D_{P}*) of the port 100, 200 and catheter 90 assembly to allow the port 100, 200 and catheter 90 assembly to pass therethrough. A longitudinal axis of the port 100, 200 and catheter 90 assembly can laterally align with a central axis 70 of the tool 420. The clinician can the advance the port 100, 200 forwards, along the central axis 70 until the port 100, 200 is seated within the housing 410 and retained therein.

In an embodiment, one of the housing cavity 412 or the handle cavity 422 can include one or more pawls, detents, or abutments configured to allow the port 100, 200 to advance forwards into the housing cavity 412, but can abut against one of the port 100, 200 or catheter 90 to mitigate retrograde movement of the port 100, 200 from the housing cavity 412. With the port 100, 200 disposed within the housing cavity 412, the catheter 90 can extend rearwards through the handle cavity opening 424. A clinician can grasp the handle 420 to manipulate the housing 410 with the port 100, 200 disposed therein. Optionally, the clinician can grasp the handle 420 and the catheter 90 together to manipulate the housing 410 and mitigate retrograde movement of the port 100, 200 from the housing cavity 412.

The clinician can then urge the front edge 430 subcutaneously. Optionally the clinician can form an incision site in skin surface for the housing 410 to pass therethrough, prior to urge the front edge 430 subcutaneously. In an embodiment, the front edge 430 of the tool can be sharpened, or can include a blade or similar structure, configured to for an incision site as the housing 410 is urged subcutaneously. In an embodiment, the blade can be formed of a different material having different mechanical properties from that of the housing 410. As the front edge 430 is urged subcutaneously the shape of the nose portion 440 can be configured to separate the subcutaneous tissues and form a tissue pocket.

When the tissue pocket is formed, the clinician can actuate the actuator 450 to transition the nose portion 440 from the closed position to the open position. With the nose portion 440 in the open position, the port 100, 200 disposed within the housing cavity 412 can advance forwards of the tool 402 and be disposed within the tissue pocket. In an embodiment, the clinician can manipulate the catheter 90 to advance the port 100, 200 forwards from the housing cavity 412, the tool 402 can then be withdrawn proximally. In an embodiment, with the nose portion 440 in the open position, the port 100, 200 can remain stationary within the tissue pocket, optionally by the clinician manipulating the catheter 90. The tool 402 can then be withdrawn proximally from the tissue pocket leaving the port 100, 200 in place. In an embodiment, as the tool 402 is withdrawn proximately, the catheter 90 can pass axially through handle cavity opening 424 and through the housing cavity opening 414 disposed along a lower surface until the tool 402 disengages with the port 100, 200 and catheter 90 assembly.

While some particular embodiments have been disclosed herein, and while the particular embodiments have been disclosed in some detail, it is not the intention for the particular embodiments to limit the scope of the concepts provided herein. Additional adaptations and/or modifications can appear to those of ordinary skill in the art, and, in broader aspects, these adaptations and/or modifications are encompassed as well. Accordingly, departures may be made from the particular embodiments disclosed herein without departing from the scope of the concepts provided herein.

## Claims

1. A port placement system (400), configured for placing a port subcutaneously, comprising:
a port (100) defining a lumen (140) extending along a longitudinal axis from an inlet (122) to an outlet (124), the lumen (140) defining a uniform cross-sectional diameter between the inlet (122) and the outlet (124);
a catheter (90) coupled to and in fluid communication with the outlet (124) of the port (100); and
a placement tool (402) comprising:
a housing (410) defining a cavity (412) configured to receive the port (100) therein, and including an elongate opening (414) extending longitudinally along a bottom surface thereof and communicating with the cavity (412);
a handle (420) extending from the housing (410); and
a nose portion (440) hingedly coupled to the housing (410) at an opposite end from the handle (420), and configured to transition between a closed position and an open position to release the port (100) from the cavity (412).

2. The port placement system according to claim 1, wherein the nose portion (440) defines a tapered outer profile.

3. The port placement system according to any one of claims 1-2, wherein the placement tool (402) includes a sharpened front edge (430) configured to form one of an incision site or a tissue pocket.

4. The port placement system according to claim 3, further including a blade disposed in the front edge (430).

5. The port placement system according to any one of claims 1-4, further including an actuator button (450) disposed on the handle (420) and configured to transition the nose portion (440) between the open position and the closed position.

6. The port placement system according to any one of claims 1-5, wherein a lateral width of the elongate opening (414) is less than a diameter of the port (100) and greater than a diameter of the catheter (90).

7. The port placement system according to any one of claims 1-6, wherein the handle (420) defines a cavity (422) and an elongate opening (424), the handle cavity communicating with the cavity (412) of the housing (410) and the handle elongate opening (424) communicating with the elongate opening (414) of the housing (410).

8. The port placement system according to claim 7, wherein a lateral width of the handle elongate opening (424) is larger than a diameter of the port (100).

9. The port placement system according to any one of claims 1-8, wherein the lumen (140) of the port (100) defines a uniform cross-sectional area.

10. The port placement system according to any one of claims 1-9, wherein the lumen (140) of the port (100) defines a uniform cross-sectional shape.

11. The port placement system according to any one of claims 1-10, wherein the lumen (140) of the port (100) includes a valve or a needle penetrable septum.

12. The port placement system according to any one of claims 1-11, wherein the outlet (124) of the port (100) is defined by a stem (120) configured to be inserted into a lumen (92) of the catheter (90).

13. The port placement system according to any one of claims 1-12, further including a cathlock (80) configured to secure the catheter (90) to the port (100).

14. The port placement system according to any one of claims 1-13, wherein the port (100) further includes a nub (150) extending from a bottom edge of the inlet (122) and configured to receive an access needle, impinging thereon, and direct the needle towards the inlet.

15. The port placement system according to any one of claims 1-14, wherein the port (100) includes an echogenic or radiopaque material, and/or
wherein an axis of the lumen (140) aligns with an axis of the catheter (90).

## Patentansprüche

1. Anschlussplatzierungssystem (400), das zum subkutanen Platzieren eines Anschlusses konfiguriert ist, umfassend:
einen Anschluss (100), der ein Lumen (140) definiert, das sich entlang einer Längsachse von einem Einlass (122) zu einem Auslass (124) erstreckt, wobei das Lumen (140) einen gleichförmigen Querschnittsdurchmesser zwischen dem Einlass (122) und dem Auslass (124) definiert;
einen Katheter (90), der mit dem Auslass (124) des Anschlusses (100) gekoppelt ist und mit diesem in Fluidverbindung steht; und
ein Platzierungswerkzeug (402), umfassend:
ein Gehäuse (410), das einen Hohlraum (412) definiert, der dazu konfiguriert ist, den Anschluss (100) darin aufzunehmen, und eine längliche Öffnung (414) einschließt, die sich in Längsrichtung entlang einer Oberfläche der Unterseite davon erstreckt und mit dem Hohlraum (412) in Verbindung steht;
einen Griff (420), der sich vom Gehäuse (410) erstreckt; und
einen Nasenabschnitt (440), der an einem dem Griff (420) gegenüberliegenden Ende gelenkig mit dem Gehäuse (410) gekoppelt ist und dazu konfiguriert ist, zwischen einer geschlossenen Position und einer offenen Position zu wechseln, um den Anschluss (100) aus dem Hohlraum (412) freizugeben.

2. Anschlussplatzierungssystem nach Anspruch 1, wobei der Nasenabschnitt (440) ein sich verjüngendes Außenprofil definiert.

3. Anschlussplatzierungssystem nach einem der Ansprüche 1-2, wobei das Platzierungswerkzeug (402) eine geschärfte Vorderkante (430) einschließt, die dazu konfiguriert ist, eine Einschnittstelle oder eine Gewebetasche zu bilden.

4. Anschlussplatzierungssystem nach Anspruch 3, weiter einschließend eine Klinge, die in der Vorderkante (430) angeordnet ist.

5. Anschlussplatzierungssystem nach einem der Ansprüche 1-4, weiter einschließend einen Betätigungsknopf (450), der auf dem Griff (420) angeordnet ist und dazu konfiguriert ist, den Nasenabschnitt (440) zwischen der offenen Position und der geschlossenen Position zu wechseln.

6. Anschlussplatzierungssystem nach einem der Ansprüche 1-5, wobei eine laterale Breite der länglichen Öffnung (414) kleiner als ein Durchmesser des Anschlusses (100) und größer als ein Durchmesser des Katheters (90) ist.

7. Anschlussplatzierungssystem nach einem der Ansprüche 1-6, wobei der Griff (420) einen Hohlraum (422) und eine längliche Öffnung (424) definiert, wobei der Hohlraum des Griffs mit dem Hohlraum (412) des Gehäuses (410) in Verbindung steht und die längliche Öffnung (424) des Griffs mit der länglichen Öffnung (414) des Gehäuses (410) in Verbindung steht.

8. Anschlussplatzierungssystem nach Anspruch 7, wobei eine laterale Breite der länglichen Öffnung (424) des Griffs größer als ein Durchmesser des Anschlusses (100) ist.

9. Anschlussplatzierungssystem nach einem der Ansprüche 1-8, wobei das Lumen (140) des Anschlusses (100) eine gleichförmige Querschnittsfläche definiert.

10. Anschlussplatzierungssystem nach einem der Ansprüche 1-9, wobei das Lumen (140) des Anschlusses (100) eine gleichförmige Querschnittsform definiert.

11. Anschlussplatzierungssystem nach einem der Ansprüche 1-10, wobei das Lumen (140) des Anschlusses (100) ein Ventil oder ein nadeldurchdringbares Septum einschließt.

12. Anschlussplatzierungssystem nach einem der Ansprüche 1-11, wobei der Auslass (124) des Anschlusses (100) durch einen Schaft (120) definiert ist, der dazu konfiguriert ist, in ein Lumen (92) des Katheters (90) eingeführt zu werden.

13. Anschlussplatzierungssystem nach einem der Ansprüche 1-12, weiter einschließend eine Katheterverriegelung (80), die dazu konfiguriert ist, den Katheter (90) an dem Anschluss (100) zu sichern.

14. Anschlussplatzierungssystem nach einem der Ansprüche 1-13, wobei der Anschluss (100) weiter eine Noppe (150) einschließt, die sich von einer Kante der Unterseite des Einlasses (122) erstreckt und dazu konfiguriert ist, eine darauf auftreffende Zugangsnadel aufzunehmen und die Nadel in Richtung des Einlasses zu lenken.

15. Anschlussplatzierungssystem nach einem der Ansprüche 1-14, wobei der Anschluss (100) ein echogenes oder strahlenundurchlässiges Material einschließt, und/oder
wobei eine Achse des Lumens (140) mit einer Achse des Katheters (90) ausgerichtet ist.

## Revendications

1. Système (400) de placement d'orifice, configuré pour placer un orifice sous-cutané, comprenant :
un orifice (100) définissant une lumière (140) s'étendant le long d'un axe longitudinal d'une entrée (122) à une sortie (124), la lumière (140) définissant un diamètre de section transversale uniforme entre l'entrée (122) et la sortie (124) ;
un cathéter (90) couplé à et en communication fluidique avec la sortie (124) de l'orifice (100) ; et
un outil (402) de placement, comprenant :
un boîtier (410) définissant une cavité (412) configurée pour recevoir l'orifice (100) en son sein, et incluant une ouverture (414) allongée s'étendant longitudinalement le long d'une surface inférieure de celle-ci et communiquant avec la cavité (412) ;
une poignée (420) s'étendant à partir du boîtier (410) ; et
une partie de nez (440) couplée de manière articulée au boîtier (410) à une extrémité opposée à la poignée (420), et configurée pour effectuer un passage entre une position fermée et une position ouverte pour libérer l'orifice (100) de la cavité (412).

2. Système de placement d'orifice selon la revendication 1, dans lequel la partie de nez (440) définit un profil extérieur effilé.

3. Système de placement d'orifice selon l'une quelconque des revendications 1 à 2, dans lequel l'outil (402) de placement inclut un bord avant (430) aiguisé configuré pour former l'un parmi un site d'incision ou une poche tissulaire.

4. Système de placement d'orifice selon la revendication 3, incluant en outre une lame disposée dans le bord avant (430).

5. Système de placement d'orifice selon l'une quelconque des revendications 1 à 4, incluant en outre un bouton (450) d'actionneur disposé sur la poignée (420) et configuré pour faire passer la partie de nez (440) entre la position ouverte et la position fermée.

6. Système de placement d'orifice selon l'une quelconque des revendications 1 à 5, dans lequel une largeur latérale de l'ouverture (414) allongée est inférieure à un diamètre de l'orifice (100) et supérieure à un diamètre du cathéter (90).

7. Système de placement d'orifice selon l'une quelconque des revendications 1 à 6, dans lequel la poignée (420) définit une cavité (422) et une ouverture (424) allongée, la cavité de poignée communiquant avec la cavité (412) du boîtier (410) et l'ouverture (424) allongée de poignée communiquant avec l'ouverture (414) allongée du boîtier (410).

8. Système de placement d'orifice selon la revendication 7, dans lequel une largeur latérale de l'ouverture (424) allongée de poignée est supérieure à un diamètre de l'orifice (100).

9. Système de placement d'orifice selon l'une quelconque des revendications 1 à 8, dans lequel la lumière (140) de l'orifice (100) définit une zone de section transversale uniforme.

10. Système de placement d'orifice selon l'une quelconque des revendications 1 à 9, dans lequel la lumière (140) de l'orifice (100) définit une forme de section transversale uniforme.

11. Système de placement d'orifice selon l'une quelconque des revendications 1 à 10, dans lequel la lumière (140) de l'orifice (100) inclut une valve ou un septum pénétrable par aiguille.

12. Système de placement d'orifice selon l'une quelconque des revendications 1 à 11, dans lequel la sortie (124) de l'orifice (100) est définie par une tige (120) configurée pour être insérée dans une lumière (92) du cathéter (90).

13. Système de placement d'orifice selon l'une quelconque des revendications 1 à 12, incluant en outre un verrou (80) de cathéter configuré pour fixer le cathéter (90) à l'orifice (100).

14. Système de placement d'orifice selon l'une quelconque des revendications 1 à 13, dans lequel l'orifice (100) inclut en outre un bossage (150) s'étendant à partir d'un bord inférieur de l'entrée (122) et configuré pour recevoir une aiguille d'accès, la frapper, et diriger l'aiguille vers l'entrée.

15. Système de placement d'orifice selon l'une quelconque des revendications 1 à 14, dans lequel l'orifice (100) inclut un matériau échogène ou radio-opaque, et/ou dans lequel un axe de la lumière (140) s'aligne avec un axe du cathéter (90).
